# EUROPEAN PATENT APPLICATION

(11) **EP 2 664 621 A1**
(43) Date of publication of application: **20.11.2013**
(21) Application number: 11855892.3
(22) Date of filing: 30.12.2011
(51) Int. Cl.: C07F 1/08, C07F 15/00, C07K 5/037, C07H 19/167, A61K 31/708, A61K 38/06, A61K 31/28, A61K 31/30, A61P 31/12

(54) **ANTIVIRAL AGENT**

(30) Priority: 11.01.2011 RU 2011101479
(71) Applicant: "Ivy Pharm" Limited Liability Company, St.Petersburg 190121 (RU)
(72) Inventor: BALAZOVSKY, Mark Borisovich, St.Petersburg 199106 (RU); ANTONOV, Viktor Georgievich, Leningradskaya Oblast Vsevolozhsk 188643 (RU); BELYAEV, Alexandr Nikolaevich, St.Petersburg 192212 (RU); EREMIN, Alexei Vladimirovich, St.Petersburg 198260 (RU)
(74) Representative: Stewart, Hazel
(86) International application number: PCT/RU2011/001056
(87) International publication number: WO 2012/096596

(57) **Abstract**

The present invention proposes a combination of bis-(gamma-L-glutamyl)-L-cysteinyl-glycine or a salt thereof, inosine and coordination compounds formed by palladium, copper and (gamma-L-glutamyl)-L-cysteinyl-glycine; a pharmaceutical composition and an anti-viral agent based on said combination, and also a method for treatment of a viral disease.

## Description

### Field of the invention

This invention relates to bioinorganic chemistry and medicine and specifically to the preparation of antiviral medicinal preparations, and may be used in medicine and veterinary medicine for the treatment of viral diseases.

### Prior art

Interferons, interleukins and nucleosides are usually used for the treatment of viral diseases. Such treatment is effective in the acute phase of a disease, but is often inadequate during the chronic course of diseases.

Interferon treatment is accompanied by the development of side effects. Adverse effects of the treatment most commonly observed include influenza-like syndrome, symptoms of gastro-intestinal and psychogenic disorders, signs of myelosuppression, and disorders of thyroid and parathyroid gland functions. Many adverse reactions of interferons are potentiated by synthetic modified analogs of nucleosides (ribavirin, vidarabine, ribomidil, etc.), which are widely used in the treatment of viral diseases.

Said treatment complications are observed in 10-42% of clinical cases of chronic hepatitis, thus such treatment can be considered as fairly dangerous as regards the risk of complications even in the conditions of specialist hospitals and at the present time there are no low-toxicity effective means for the treatment, in particular, of chronic viral hepatitis, thus the creation of these is one of the urgent tasks of modern pharmacological and medical science.

Patents RU2153350 and RU2153351 describe a pharmacological solution for potentiating the anti-viral activity of inosine, preferably by using a coordination compound of cisplatin and oxidized glutathione. However, cisplatin is a complex compound of platinum (Pt), the use of which is associated with the danger of toxic and mutagenic effects, so that the use of a drug containing it is restricted.

### Substance of the invention

The object of this invention is to create a wide-spectrum anti-viral agent which is effective during the chronic course of diseases and has minimal side effects.

Said object is achieved in that a combination of bis-(y-L-glutamyl)-L-cysteinyl-glycine or a salt thereof, inosine and coordination compounds formed by palladium, copper and (γ-L-glutamyl)-L-cysteinyl-glycine is proposed. Said combination is referred to further as "the agent according to the invention".

The bis-(γ-L-glutamyl)-L-cysteinyl-glycine in this combination is preferably provided in the form of the disodium salt.

It is desirable that the bis-(γ-L-glutamyl)-L-cysteinyl-glycine disodium salt:inosine: palladium:copper molar ratio is in the range 100-10000:100-10000:1-10:1-10, for example 1000:1000:1:1.

It is desirable that cis-diaminodichloropalladium be selected as the source of palladium, and copper dichloride as the source of copper.

In one embodiment of the invention, the combination may comprise a catalyst based on palladium cis-diaminodichloride, copper dichloride and (y-L-glutamyl)-L-cysteinyl-glycine, prepared in situ.

In an alternative embodiment, such a catalyst may be prepared in advance.

The proposed combination exhibits anti-viral activity, and can be used in the treatment of infectious, and in particular viral, diseases.

Also proposed is a pharmaceutical composition which comprises said combination and a pharmaceutically acceptable excipient.

Such a pharmaceutical composition exhibits anti-viral activity, and can be used in the treatment of infectious, and in particular viral, diseases.

Also proposed is an anti-viral agent which comprises a solution of said combination in acetate buffer.

It is desirable that the bis-(γ-L-glutamyl)-L-cysteinyl-glycine disodium salt:inosine: palladium:copper molar ratio in said agent is in the range 100-10000:100-10000:1-10:1-10, for example 1000:1000:1:1.

This agent may be used for the treatment of a virus selected from the group which includes influenza virus, hepatitis C virus, hepatitis B virus, tick-borne encephalitis virus, Rift Valley fever virus and Venezuelan equine encephalitis virus.

Also proposed is a method for the treatment of a viral disease in a patient in need thereof, in which an effective amount of said combination, composition or agent is administered to the patient. The viral disease may be selected from the group which includes influenza, hepatitis C, hepatitis B, tick-borne encephalitis, Rift Valley fever and Venezuelan equine encephalitis.

### Description of the graphic materials

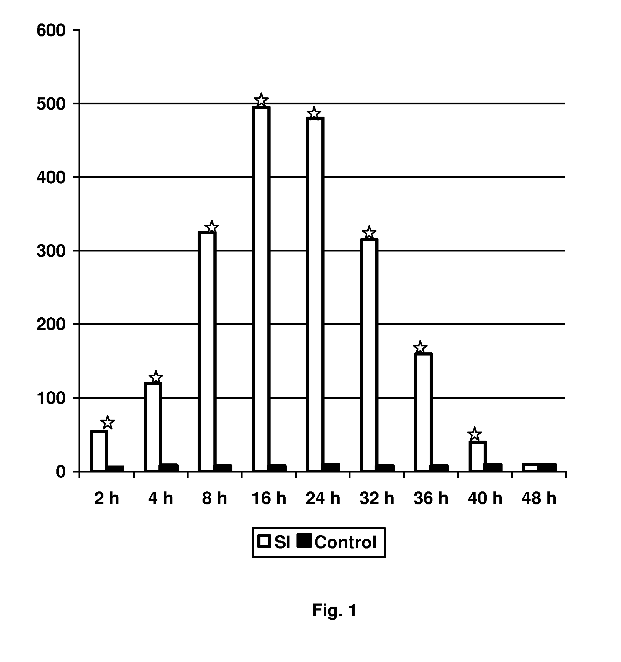

Figure 1 shows a typical profile of change in the dynamics of IFN-α in the blood serum of experimental animals after a single administration of the agent according to the invention. The blood serum IFN-α level (U/ml) is shown on the Y axis. Stars denote statistically reliable differences as compared with the control at p<0.05.

### Detailed description of the invention

The agent according to the invention exhibits an anti-viral effect and also an interferonogenic effect.

Inosine is 1,9-dihydro-9-beta-D-ribofuranosyl-6H-purin-6-one (ribofuranosyl-hypoxanthine.

Inosine is an anabolic agent, a stimulator of metabolic processes and a precursor of ATP. It increases the energy balance, and improves coronary blood circulation and metabolic processes in the myocardium. It exhibits an anti-hypoxic effect. It is employed in IHD (myocardial infarction, stenocardia), cardiomyopathy, cardiac rhythm disorders, myocarditis, myocardiodystrophy, diseases of the liver (hepatitis, cirrhosis of the liver, fatty dystrophy of the liver), gastric and duodenal ulcer, and open-angle glaucoma.

Glutathione, gamma-glutaminyl-cysteinyl-glycine, is a tripeptide, formed by residues of three amino acids: glutamic acid, cysteine and glycine.

Glutathione is present in all living organisms and is of great importance for oxidation-reduction reactions because of the ability of the sulfhydryl group (SH-) of cysteine to enter into the reversible reaction:

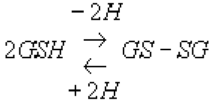

In this description, reduced glutathione (GSH) denotes (γ-L-glutamyl)-L-cysteinyl-glycine. The oxidized form of glutathione (GSSG) denotes bis-(γ-L-glutamyl)-L-cysteinyl-glycine.

If not otherwise indicated, glutathione is to be understood as reduced glutathione in this description.

Glutathione is able to form salts with cations, in particular Na.

A combination according to the invention contains d-metals. The sources of the d-metals, palladium and copper, may be various salts thereof which on dissolving in water lead to the formation of complex compounds. In particular, when simple salts of copper (II), such as CuCl₂ or CuBr₂, are added to an aqueous solution, the aquation thereof occurs, accompanied by subsequent hydrolysis and formation of oligonuclear aquahydroxy-complexes of copper (II) in the solution. Palladium can also be added in the form of suitable salts thereof, in particular cis-diaminodichloropalladium.

The reaction for preparation of the agent according to the invention GSSGNa₂/inosine/Pd/Cu = 1000/1000/1/1 corresponds to the equation:

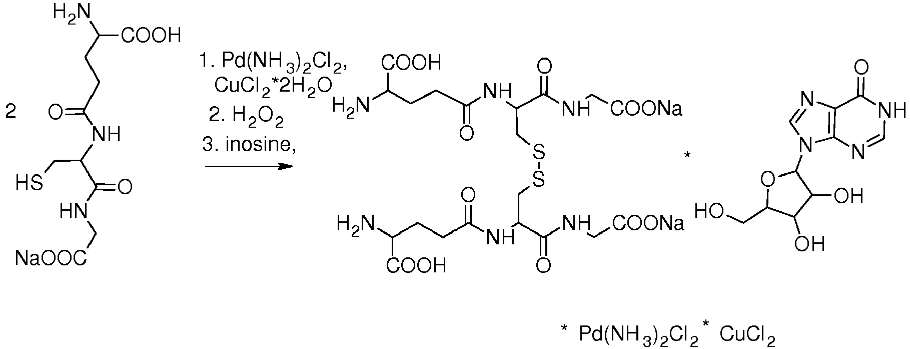

Preparation of the agent according to the invention can be performed by several methods.

One of the embodiments of the process for preparing the agent according to the invention comprises preliminary preparation of a palladium-copper catalyst (coordination compounds of palladium and copper and γ-L-glutamyl-L-cysteinyl-glycine) (stage 1, example 1) with subsequent addition of the resultant catalyst solution to the reaction mass, performing oxidation, mixing the resultant solution with a solution of inosine, filtering and freeze-drying.

In another embodiment, a variant is proposed for *in situ* preparation of a palladium-copper catalyst (example 2), followed by oxidation, mixing the resultant solution with a solution of inosine, filtering and freeze-drying.

In a third embodiment (example 3), a separate preparation variant is proposed with introduction of an additional stage of vacuum-sublimation drying (freeze-drying) of a solution of the disodium salt of bis-(γ-L-glutamyl)-L-cysteinyl-glycine (containing coordination compounds of palladium and copper), subsequently dissolving the freeze-dried material, mixing with a solution of inosine, filtering and freeze-drying the solution of the agent according to the invention.

The final embodiment, although more costly in terms of energy and materials, has an advantage associated with the absence of secondary processes, namely inosine oxidation processes.

One of the most important functions of palladium during the catalytic oxidation of thiols RSH is the formation of coordination compounds - products of the addition of thiolate ions RS⁻ to the palladium ion, with subsequent oxidation thereof and breakdown of the coordination polyhedron.

Experimental investigations of catalytic systems employing copper-palladium catalysts demonstrate the significantly greater catalytic efficiency of these as compared to binuclear thiolate-bridge compounds of palladium(II). In contrast to aqueous solutions of oxidized glutathione GSSG, containing thiolate complexes of copper Cu^{I}ₖ(SR)ₘ, aqueous solutions containing Pd-Cu are, according to ¹H NMR, IR spectroscopy and HPLC data, resistant to decomposition processes.

Investigations show that the Pd:Cu ratio in Pd-Cu catalysts lies optimally in the range from 1:0.2 to 1:2.

For processes of mild selective oxidation of GSH to GSSG, the conclusion can be drawn that binuclear thiolate bridge complexes of palladium(II) fulfill the basic function of oxidation catalysts, while thiolate complexes of copper(I) should be regarded as chemical sites which modify the catalytic activity thereof or, in other words, control the catalytic activity thereof.

A combination of functional binuclear palladium coordination compounds, for example with the formula [Pd₂^{II}(µ-SR)₂(NH₃)₄], with bidentate-bridge coordination of glutathione with a control site of {Cu^{I}ₖ(SR)ₘ} type, formed from copper salts CuX₂ (where X= Cl⁻ or Br⁻), converted in a medium of thiols into the corresponding thiolate derivatives of copper(I) compounds, thus discloses one of the most effective approaches to controlling the activity of catalytic systems based on complexes of Pd₂^{II} and Cu^{I}.

The formation of a complex compound which includes Cu, Pd and GSH in comparable amounts provides the biological effect of the proposed combination.

Pharmaceutically acceptable excipients are used in order to prepare pharmaceutical compositions according to the invention. In particular, these are inorganic or organic vehicles. Lactose, corn starch or derivatives thereof, talc, stearic acid or salts thereof, etc. may be used, for example as such vehicles for tablets, coated tablets, pills and hard gelatin capsules. Suitable vehicles for soft gelatin capsules are, for example, vegetable oils, waxes, fats, semi-solid and liquid polyols, etc. Suitable vehicles for the preparation of solutions and syrups are, for example, water, polyols, sucrose, invert sugar, glucose, etc. Suitable vehicles for suppositories are, for example, natural or hardened oils, waxes, fats, semi-solid or liquid polyols, etc.

Furthermore, pharmaceutical compositions may contain preservatives, solubilizers, stabilizers, wetting agents, emulsifiers, sweeteners, colorants, correctives, salts to regulate the osmotic pressure, buffers, masking agents or antioxidants and other necessary components.

In general, all products and methods according to the invention may alternatively include, consist of or essentially consist of any suitable components and stages disclosed in this description or known from the prior art to one skilled in the art, and also products or methods according to the invention may additionally or alternatively exclude any particular component, or stage, or object, which is used in a product or method known from the prior art, or which is not necessary in order to achieve the technical result of this invention.

### EXAMPLES

The invention is further explained by specific examples.

### Example 1. Preparation of the agent according to the invention

**Stage 1.** 20 mg (94.6 µmol) of cis-[Pd(NH₃)₂Cl₂] is dispersed cold in 20 ml of distilled water, then 30 mg of GSH (97.6 µmol) is added to the resultant suspension and stirred until a homogeneous light-yellow solution is obtained.

**Stage 2.** 5 ml of a solution containing 14.52 mg (85.2 µmol) of copper(II) chloride dihydrate is added to the previously prepared reaction mixture. The pH of the resultant yellow-green solution of catalyst is adjusted to 5.5-6.0 with 0.01 M sodium hydroxide solution.

**Stage 3.** 58.19 g (0.189 mol) of GSH are weighed in a glass beaker, then 200 ml of distilled water are added while stirring and cooled to 10-15°C. 7.57 g of NaOH (0.189 mol) are separately dissolved in 50-60 ml of distilled water and the resultant solution is added to the suspension of GSH while stirring vigorously and not allowing the reaction mass to heat to more than 15-20°C, continuing the stirring until a homogeneous translucent solution is formed. The pH of the reaction mixture is adjusted to 5.5-6.0 with 0.01 M sodium hydroxide solution if necessary. A previously prepared catalyst solution is added to the resultant reaction system, the beaker is transferred to an ice bath (5-10°C) and 100-102 ml (∼ 0.1 mol) of freshly-prepared 1 M hydrogen peroxide solution is added in small portions over 45-60 min. while stirring vigorously. HPLC is used to check that the reaction proceeds to completion.

25.38 g (0.095 mol) of ribofuranosylhypoxanthine (inosine) is dissolved separately in 150 ml of hot (60-70°C) distilled water. After dissolving completely, the solution is cooled to room temperature and added to the reaction mixture. After sterilizing filtration, the solution is frozen and subjected to vacuum-sublimation (freeze) drying.

The GSSG-inosine-palladium-copper molar ratio in the resultant preparation is 1000-1000-1-0.9.

### Example 2. Preparation of the agent according to the invention

330 g (1.074 mol) of reduced glutathione is suspended in 3100 ml of water. 42.96 g (1.074 mol) of sodium hydroxide, in the form of a 16% aqueous solution, are added to the resultant suspension while stirring continuously and cooling. 0.1135 g (0.537 mmol) of cis-diaminodichloropalladium and 0.0915 g (0.537 mmol) of copper chloride dihydrate are added to the resultant solution, and 304.5 g of 6% hydrogen peroxide solution (0.537 mol) are added while stirring thoroughly and monitoring the temperature (not more than 15°C). After adding the peroxide, the resultant solution is left to stand at room temperature for 1.5 hours. 144 g (0.537 mol) of inosine dissolved in the minimum amount of water are added to the resultant solution of oxidized glutathione, filtered through a 0.22 µ filter and freeze-dried. The yield of bis-(γ-L-glutamyl)-L-cysteinyl-glycine ribofuranosyl-hypoxanthine-disodium:cis-diaminodichloropalladium: copper dichloride (in 1000:1:1 ratio) is 99%.

### Example 3. Preparation of the agent according to the invention

### Stage 1

330 g (1.074 mol) of reduced glutathione are suspended in 3100 ml of water. 42.96 g (1.074 mol) of sodium hydroxide, in the form of a 16% aqueous solution, are added to the resultant suspension. The reaction mass is then cooled and 0.1135 g (0.537 mmol) of cis-diaminodichloropalladium and 0.0915 g (0.537 mmol) of copper chloride dihydrate are added. 304.5 g of 6% hydrogen peroxide solution (0.537 mol) are added to the resultant solution while checking the temperature (not more than 15°C). The resultant solution is left to stand at room temperature for 1.5 hours. It is then filtered through a 0.22 p filter and freeze-dried. 365 g of the disodium salt of bis-(γ-L-glutamyl)-L-cysteinyl-glycine are obtained (content of water 4%, Pd - 57 mg, Cu - 34 mg).

### Stage 2

The freeze-dried disodium salt of bis-(γ-L-glutamyl)-L-cysteinyl-glycine from the previous stage is dissolved in the minimum amount of water and added to a solution of inosine (144 g, 0.537 mol) in water. The resultant solution is filtered through a 0.22 p filter and freeze-dried. The yield for the two stages is 98%.

### Example 4. Preparation of a medicinal form of the agent according to the invention

13.6 g of sodium acetate trihydrate and 60 g of the substance of the agent according to the invention are added to 0.8 l of water for injection in a solution preparation vessel with a capacity of 1 l and stirred until dissolved, after which the total volume is adjusted to 1.0 l. The pH value is determined using a pH meter and 20% acetic acid is added to pH 6.0±0.2. Sterilizing filtration is performed using a sterilizing filter with a pore size of 0.22 p and it is filled into glass ampoules (1 ml - 1 ampoule). As a result, 970 ampoules are obtained (allowing for losses), which contain a composition for intravenous or intramuscular injection, comply with the requirements of normative documentation for 3 years and have the following composition:

| | |
|---|---|
| Agent according to the invention | 60 g |
| Sodium acetate trihydrate | 13.6 g |
| Acetic acid | to pH 6.0 |
| Water | to 1000 ml |

### Example 5. Therapeutic action in vivo of the agent according to the invention in the treatment of diseases caused by pathogenic DNA and RNA viruses

Inosine is characterized by a wide spectrum of pharmacological activity, including an anti-viral effect, which can be enhanced by combining it with other biologically active molecules. Patents RU2153350 and RU2153351 disclose a pharmacological solution relating to potentiation of the anti-viral effect of inosine, preferably using a coordination compound of cisplatin and oxidized glutathione. The potentiating effect is achieved due to the ability of the coordination compound of cisplatin and oxidized glutathione to catalyze a complex of reactions of oxidative modification of the target, enhancing the sensitivity thereof to the action of inosine. If the mechanism of potentiating the anti-viral effect of inosine is associated with the catalytic activity of coordination compounds, then the agent according to the invention should exhibit a similar, more marked, effect, since its composition includes a coordination compound with higher catalytic activity as compared to a coordination compound of cisplatin and oxidized glutathione.

The object of the investigation is to determine the therapeutic effectiveness of the agent according to the invention in the treatment of various viral infections.

### Compounds tested

bis-(γ-L-glutamyl)-L-cysteinyl-glycine ribofuranosylhypoxanthine-disodium:cis-diaminodichloropalladium:copper dichloride (in 1000:1:1 ratio) (the agent according to the invention)

bis-(γ-L-glutamyl)-L-cysteinyl-glycine ribofuranosylhypoxanthine-disodium:cis-diaminodichloroplatinum (reference 1)

Arbidol medicinal form, series 970609 (reference 2)

### Experimental model

- *Venezuelan equine encephalitis (VEE) virus* -pathogenic strain Trinidad. Virus-containing material for subsequent infection of laboratory animals was accumulated using 9-11 day developing chick embryos (30-50). Five sequential ten-fold dilutions of virus-containing suspension were first prepared. 0.2 ml of each dilution of virus-containing suspension was introduced into the allantoic cavity of developing chick embryos. The virus-containing suspension injection site was sealed with melted paraffin. The developing chick embryos were then placed in a thermostat at a temperature of (37±0,5)°C for 18 h, and their viability was periodically assessed using an ovoscope. On expiry of the incubation time, the viability of the developing chick embryos in the thermostat was assessed and a 10% suspension of virus-containing material was prepared from the "carcasses" of the live embryos using physiological saline with addition of antibiotics (penicillin at a rate of 100 units per 1 ml, streptomycin at 200 units per 1 ml). The resultant suspension was centrifuged for 10 min. at 1.5-2.0 thousand rev/min and a temperature of plus (3±0.5)°C. The supernatant liquid was dispensed into 1.0 ml vials and used for subsequent infection of experimental animals (mice). The initial virus titer was 10⁷-10⁸ LD₅₀/ml.
- *Rift Valley fever (RVF) virus -* pathogenic strain 8-87. Virus-containing material for infection of laboratory animals was accumulated using 3-5 day suckling mice (10-15 animals). Five sequential ten-fold dilutions of virus-containing material were first prepared. 0.02 ml of each dilution was injected into the brain of suckling mice and these were observed for 24-48 h, at the end of which the animals were sacrificed with ether, and the brain was extracted and placed three samples in each in penicillin vials which were stored in a freezer at a temperature of minus (20±0.5)°C. A 10% brain suspension was used subsequently as the virus-containing material. The initial virus titer was 10⁵-10⁶ LD₅₀/ml;
- *tick-borne encephalitis (TE) virus*, pathogenic strain Absetarov. Virus-containing material for infection of laboratory animals was accumulated using suckling mice. Five sequential ten-fold dilutions were first prepared of virus-containing material which comprised the centrifugate of a 10% suspension of the brain of previously infected mice or virus-containing material rehydrated from the freeze-dried state. 0.02 ml of each dilution was injected into the brain of suckling mice and these were observed for 24-48 h, at the end of which the animals were sacrificed with ether, and the brain was extracted and placed three samples in each in penicillin vials which were stored in a freezer at a temperature of minus (20±0.5)°C. A 10% brain suspension was used subsequently as the virus-containing material. The initial virus titer was 10²-10³ LD₅₀/ml.
- *influenza A virus strain A*/*Aichi*/*02*/*68 (H₃N₂).* Virus-containing material was obtained using 9-11 day-old developing chick embryos. Subsequent accumulation of virus-containing material for infection of laboratory animals was carried out using 5 mice, which were infected with virus-containing allantoic fluid containing the pathogenic strains A/Aichi/02/68 (H₃N₂). Three days after infection, their lungs were isolated and homogenized in a 10-fold volume of sterile physiological saline, after which the infectious activity of the virus in the homogenate was determined in a separate experiment by lethality titration in animals. The virus titer was calculated using the method of Reed and Muench (Am.J.Hyg., 1938, 27:493-497).

Studies to assess the effectiveness of the agent according to the invention were carried out on non-inbred male white mice weighing 16-18 g (360 animals).

The LD₅₀ for each causative agent was determined in non-inbred white mice, calculating the criterion using the Kerber method as modified by I.P. Ashmarin and A.A. Vorob'yev.

The effectiveness of the preparations studied was determined by comparing the values of the survival rate indices of animals in the experimental (which were given the corresponding preparations) and the control groups. The percentage of animals surviving in the experimental and control groups was determined using the tables of V.S. Genes. The infected animals were observed for 21 days and the number of living and dead animals in the experimental and control groups was recorded daily.

### Methods for statistical treatment of the results

Statistical treatment of the experimental results was carried out on a personal computer, using special programs implementing conventional statistical methods.

### Results of studying the anti-viral activity of the agent according to the invention

In all the experimental models of especially dangerous viral infections (EDVI) and influenza, the effectiveness of the agent according to the invention and comparative preparations was evaluated when they were employed using two schemes:
- 24 h before infection, at the same time as infection and 24 h, 48 h and 72 h after infection (scheme No. 1 - emergency prophylaxis);
- at the same time as infection and 24 h, 48 h and 72 h after infection (scheme No. 2 - early etiotropic treatment).

The agent according to the invention and reference 1 were injected subcutaneously in an amount of 0.5 ml in a single dose of 30 mg/kg of body weight (10 µg/mouse).

The reference preparation used when treating an influenza infection was Arbidol medicinal form, series 970609 (reference 2), which was administered 1 h after infection and then perorally once daily in a dose of 60 mg/kg over five days.

### Effectiveness of the agent according to the invention in experimental viral infection with Venezuelan encephalomyelitis

The results of the investigation are shown in Table 1.

**Table 1**

| Effectiveness of the agent according to the invention as a preparation for emergency prophylaxis and etiotropic treatment of an experimental viral infection with Venezuelan encephalomyelitis | | | | | |
|---|---|---|---|---|---|
| Preparation | Preparation administration scheme No. | Infecting dose of causative agent (number of LD₅₀) | Number of animals in group | Number of animals dead, % | Number of animals surviving, % |
| agent according to the invention | 1 | 12 | 10 | 0 | 100* |
| | | 2 | 10 | 0 | 100* |
| | 2 | 12 | 10 | 0 | 100* |
| | | 2 | 10 | 0 | 100* |
| reference 1 | 1 | 12 | 10 | 60 | 40 |
| | | 2 | 10 | 40 | 60 |
| | 2 | 12 | 10 | 50 | 50 |
| | | 2 | 10 | 40 | 60 |
| Infection control | Not administered | 12 | 10 | 100 | 0 |
| | Not administered | 2 | 10 | 100 | 0 |
| * - differences with control statistically reliable at p<0.05. | | | | | |

The results presented in Table 1 show that the preparations evaluated exhibited a protective effect in relation to experimental VEE. The agent according to the invention proved the most effective in these conditions. Here, if the preparation was used in accordance with scheme 1 (24 h before infection, at the same time as infection and 24 h, 48 h and 72 h after infection), then irrespective of the infecting dose of VEE virus the survival rate of infected mice was at a level of 100%, against 100% mortality in the control. If, however, the preparation was used in accordance with scheme 2 (at the same time as infection and 24 h, 48 h and 72 h after infection), then in these conditions, depending on the infecting dose of the causative organism, the protective effect was at a level of 100% (on infection with the causative organism in a dose of 12 LD₅₀) and 100% (on infection with the causative organism in a dose of 2 LD₅₀). When using reference 1, the indices of protection were 40-60% lower, depending on the infecting dose of the causative organism, than when using the agent according to the invention.

Thus, on the basis of the investigations carried out it can be concluded that of the preparations evaluated the agent according to the invention proved the most effective in relation to experimental VEE infection. When employed in accordance with an emergency prophylaxis scheme, the agent according to the invention provided 100% protection to the infected animals compared to 100% mortality in the control.

### Effectiveness of the agent according to the invention in experimental viral infection with Rift Valley fever.

The results of the investigation are shown in Table 2.

**Table 2**

| The effectiveness of an agent according to the invention as a preparation for emergency prophylaxis and etiotropic treatment of an experimental viral infection with Rift Valley fever | | | | | |
|---|---|---|---|---|---|
| Preparation | Preparation administration scheme No. | Infecting dose of causative agent (number of LD₅₀) | Number of animals in group | Number of animals dead, % | Number of animals surviving, % |
| Agent according to the invention | 1 | 12 | 10 | 0 | 100* |
| | | 2 | 10 | 0 | 100* |
| | 2 | 12 | 10 | 0 | 100* |
| | | 2 | 10 | 0 | 100* |
| Reference 1 | 1 | 12 | 10 | 40 | 60 |
| | | 2 | 10 | 40 | 60 |
| | 2 | 12 | 10 | 70 | 30 |
| | | 2 | 10 | 45 | 55 |
| Infection control | Not administered | 12 | 10 | 100 | 0 |
| | Not administered | 12 | 10 | 100 | 0 |
| * - differences with control statistically reliable at p<0.05. | | | | | |

The results presented in Table 2 show that the best protective and therapeutic effect in relation to RVF was obtained when using the agent according to the invention. Irrespective of the administration scheme, the preparation provided 100% protection to the infected mice as compared to 100% mortality in the control. The effectiveness of reference 1 in these conditions was low.

### Effectiveness of the agent according to the invention in experimental viral infection with experimental tick-borne encephalitis.

The results of the investigation are shown in Table 3.

**Table 3**

| The effectiveness of an agent according to the invention as a preparation for emergency prophylaxis and etiotropic treatment of an experimental viral infection with experimental tick-borne encephalitis | | | | | |
|---|---|---|---|---|---|
| Preparation | Preparation administration scheme No. | Infecting dose of causative agent (number of LD₅₀) | Number of animals in group | Number of animals dead, % | Number of animals surviving, % |
| Agent according to the invention | 1 | 12 | 10 | 0 | 100* |
| | | 2 | 10 | 0 | 100* |
| | 2 | 12 | 10 | 0 | 100* |
| | | 2 | 10 | 0 | 100* |
| Reference 1 | 1 | 12 | 10 | 20 | 80* |
| | | 2 | 10 | 0 | 100* |
| | 2 | 12 | 10 | 20 | 80* |
| | | 2 | 10 | 20 | 80* |
| Infection control | Not administered | 12 | 10 | 100 | 0 |
| | Not administered | 2 | 10 | 100 | 0 |
| * - differences with control statistically reliable at p<0.05. | | | | | |

As the results presented show, when used in animals infected with the causative organism in a dose of 2 LD₅₀ all the preparations evaluated exhibited virtually the same protective effectiveness, providing a survival rate of 80-100% of the infected mice as compared to 100% mortality in the control. At the same time, if the preparations were used in animals infected with the causative organism in a dose of 12 LD₅₀, then in these conditions the agent according to the invention exhibited a more pronounced effect.

### Effectiveness of the agent according to the invention in experimental viral infection with influenza (pathogenic strain A/Aichi/02/68).

The results of the investigation are shown in Table 4.

**Table 4**

| Effectiveness of the agent according to the invention as a preparation for emergency prophylaxis and etiotropic treatment of an experimental viral infection with influenza (pathogenic strain A/Aichi/02/68). | | | | | | |
|---|---|---|---|---|---|---|
| Preparation | Dose of preparation, mg/kg | Scheme | Death rate | Protection against death (%) | MST, days | Increase in MST, days |
| Agent according to the invention | 30 | -24, +1, +24, +48, +72 | 12/15 | 20,0 | 8,5 | 1,8 |
| | | +1, +24, +48,+72 | 9/15 | 40,0 | 11.2 | 3,1 |
| Arbidol 970609 | 60 | + 1 h and then for 5 days | 10/15 | 33,3 | 9,3 | 2,6 |
| Control - dose (without preparation) | | | 15/15 | - | 6,7 | - |
| Control - placebo (without preparation) | | | 0/15 | - | 14,0 | - |

The results obtained show that the agent according to the invention has higher specific activity than Arbidol in relation to influenza infection in white mice infected intranasally with influenza virus strain A/Aichi/02/68 (H3N2).

### Conclusion

The overall results of the investigations carried out permit the conclusion that the agent according to the invention is characterized by a prophylactic and therapeutic effect in relation to various viral diseases caused by both DNA and RNA viruses.

### Example 6. Determination of the ability of the agent according to the invention to induce interferon production

The therapeutic efficacy of the agent according to the invention in relation to various viral diseases, caused by both DNA and RNA viruses, may, inter alia, be due to its ability to increase the endogenous production of interferon, a biologically active substance which has marked anti-viral activity.

Object of the investigation: to determine the ability of the agent according to the invention to stimulate production of interferon.

Test compound: bis-(γ-L-glutamyl)-L-cysteinyl-glycine ribofuranosylhypoxanthine-disodium:cis-diaminodichloropalladium:copper dichloride (in 1000:1:1 ratio) (the agent according to the invention)

### Experimental method

The interferonogenic properties of the agent according to the invention were evaluated by titration of interferon alpha (IFN-alpha) in the blood serum of experimental animals which had received the agent according to the invention in a dose of 30 mg/kg, which the results of experiments show to be effective (see Example 3). The preparation was administered subcutaneously in a single dose. Subcutaneous administration of a placebo (0.85% sodium chloride solution) was employed in a control group of mice. Blood was collected from the mice of the experimental group (which had received one of the test preparations) and the control group in sterile conditions from the retro-orbital sinus 2, 4, 8, 16, 24, 32, 36, 40 and 48 h after injection of the preparation. The blood from five mice was combined in one sample. The dynamics of interferon accumulation was studied in sera prepared by the standard method. A culture of L-929 cells (a passaged culture of murine fibroblast cells), 153rd passage, obtained from the Institute of Cytology of the Russian Academy of Sciences (St. Petersburg) was used to titrate IFN-alpha activity in the blood serum of the experimental animals. IFN-alpha in the prepared samples was titrated using a micro-method. A culture of L-929 cells, diluted with culture medium to the required concentration (3-4 10⁵ cells/ml) was placed in the wells of 96-well plates and incubated in a thermostat for 24 h. The cell cultures were incubated in identical conditions during the entire titration process: a temperature of plus 37°C in an atmosphere with 5% CO₂ and 98% humidity. After a cell monolayer had formed on the base of the wells, the culture medium was removed from the plates and a maintenance medium was introduced, in which successive two-fold dilutions of the test samples were performed (in paired wells). On completion of 24 hours incubation, the test samples were removed and EMC test virus in working dilution (100 CPD₅₀) was introduced. The results were determined after 24 hours using an inverted microscope. The IFN titer is the value inverse to the last serum dilution in which 50% protection against test virus CPD is observed.

### Results of the investigation

The dynamics of changes in IFN-alpha in the blood serum of experimental animals after a single administration of the agent according to the invention are presented in Fig. 1.

The results obtained show that the IFN-alpha level started to increase two hours after administration of the agent according to the invention by a factor of 5-6, reached a maximum after 16-24 hours, exceeding the background values by a factor of 45-50, and returned to normal values by 48 hours after administration of the agent according to the invention. The dynamics of change in IFN-alpha level demonstrate that the agent according to the invention has interferonogenic activity. Endogenous IFN-alpha has a definite advantage over preparations of exogenous interferon: when interferonogens are introduced into the body, interferon is produced which does not exhibit antigenicity; the adverse effects which are inherent to preparations of exogenous IFN-alpha do not occur; the synthesis of induced IFN-alpha in the body is balanced and is subject to the control and regulatory mechanisms (repressor-translation) which ensure protection of the body against interferon overload; a single administration of interferonogens provides relatively prolonged circulation of endogenous IFN-alpha. The action of the agent according to the invention corresponds to the above-mentioned effects of interferonogens.

The agent according to the invention is thus characterized by the ability to enhance the production of interferon-alpha.

### Example 7. Therapeutic effectiveness of the agent according to the invention-GSSGNa₂/inosine/Pd/Cu in chronic viral hepatitis C (CVHC)

Female patient No. 1: 48 years of age.

Diagnosis: CVHC, replication phase (PCR HCV+ - 3.60x10⁷ copies/ml = 9.00x10⁶ IU/ml), moderate level of activity. Chronic autoimmune thyroiditis. Hypothyrosis.

### Duration of illness 20 years

History shows intolerance to specific anti-viral therapy, including pegylated interferon, ribavirin and other anti-viral preparations, and interferon inducers. Takes L-thyroxin regularly, 150 mg once daily in the morning.

Complains of weakness, a feeling of heaviness in the right hypochondrium, an aftertaste, lack of appetite, poor sleep and irritability.

During the last five years has frequently noted the appearance of aching pains in the right hypochondrium, weakness and periodic alteration in the color of the urine.

After examination (for results see Table 5, column 3), treatment was carried out in a day-patient department with a medicinal form of the agent according to the invention, given at 60 mg intramuscularly 3 times per week, with a two-day break (Saturday, Sunday).

**Table 5.**

| Dynamics of change in the values of laboratory diagnostic criteria during treatment with a medicinal form of the agent according to the invention | | | | | |
|---|---|---|---|---|---|
| Criterion | Reference value | Before treatment | After 4 weeks | After 12 weeks | After 24 weeks |
| 1 | 2 | 3 | 4 | 5 | 6 |
| Viral load PCR HCV (copies/ml) | - | 9.60x10⁷ | 3.03 x10⁶ | 1.42 x10⁶ | 2.65 x10³ |

| Biochemical analysis of blood | | | | | |
|---|---|---|---|---|---|
| ALT (U/L) | 0-45 | 194 | 156 | 95 | 64 |
| AST (U/L) | 5-34 | 206 | 198 | 112 | 83 |
| GGTP (U/L) | 9-36 | 216.75 | 186.24 | 164.65 | 96.20 |
| ALP (U/L) | Up to 240 | 302 | 298 | 246 | 180 |
| Total bilirubin (mg/dl) | 0.2-1.2 | 6.45 | 3.31 | 1.45 | 0.96 |
| Direct bilirubin (mg/dl) | 0-0.5 | 4.45 | 1.52 | 1.02 | 0.44 |
| Total protein (g/dl) | 6.4-8.3 | 6.0 | 6.3 | 6.7 | 7.4 |
| Albumin (g/dl) | 3.2-5.0 | 3.2 | 3.5 | 3.7 | 4.2 |
| Globulins (g/dl) | 2.7-3.3 | 2.8 | 2.7 | 3.0 | 3.0 |
| Alb./Glob. | 1.2-2.0 | 1.14 | 1.3 | 1.23 | 1.4 |
| Ferritin (ng/ml) | 6-223 | 446.73 | 394.66 | 356.07 | 256.21 |
| Blood iron µg/dl | 25-156 | 201.2 | 200.07 | 184.27 | 159.87 |

| Blood analysis | | | | | |
|---|---|---|---|---|---|
| Erythrocytes x10¹²/L | 4.0-5.6 | 3.58 | 3.67 | 4.98 | 4.82 |
| Hemoglobin (g/dl) | 12-16 | 8.7 | 9.3 | 10.1 | 11.8 |
| Hematocrit (%) | 27-31 | 21 | 27 | 27 | 31 |
| Leukocytes x10⁹/L | 4-11 | 3.6 | 3.62 | 4.8 | 5.2 |
| Neutrophils (%) | 45-70 | 40 | 44.9 | 50 | 56 |
| Basophils (%) | 0.5-1 | 0 | 0.6 | 0.8 | 1 |
| Eosinophils (%) | 1-5 | 1 | 2.4 | 2.2 | 2 |
| Monocytes (%) | 1-8 | 4 | 4.3 | 4 | 3 |
| Lymphocytes (%) | 25-40 | 55 | 48.1 | 43 | 38 |
| Thrombocytes (x10⁹/l) | 180-320 | 90.2 | 133 | 159 | 212 |

| Blood coagulation system | | | | | |
|---|---|---|---|---|---|
| Prothrombin time (s) | 10-12.5 | 17.3 | 15.3 | 14.1 | 11.1 |
| Activated partial prothrombin time (s) | 20-36 | 42 | 40 | 36 | 30 |
| Thrombin time (s) | 10-13.5 | 17.6 | 16.0 | 13.9 | 12.8 |
| Partial thrombin time (s) | 10-12 | 17.4 | 16.1 | 12.7 | 11.9 |
| Bleeding time (min.) | less than 4 min. | 8 | 8 | 6 | 4 |
| Blood clotting time (min.) | 5-10 min. | 9 | 9 | 8,3 | 8 |

The medicinal form of the agent according to the invention is well tolerated. After injection, the patient noted a surge of energy and improvement in mood. An objective criterion of the response to treatment is the dynamics of change in the viral load, which had fallen three-fold after a month, by a factor of 60 after three months, and by more than four logarithms after six months of treatment. The reduction in viral load was accompanied by a fall in the intensity of cytolysis reactions (normalization of transaminase by the end of treatment), and restoration of the physiologically optimal functional activity of hepatocytes (normalization of blood protein and the values of the indices of pigment and other types of metabolism). The therapeutic effect of the preparation led to normalization of the cellular composition of the blood and coagulation system activity.

On examination, the liver extended for 0.5 cm from under the costal arch. The edge of the liver was soft and not tender.

Among the particular features of the treatment, it must be noted that the dose of L-thyroxin needed to be reduced every three weeks. By the end of the treatment the dose of L-thyroxin had been halved.

Use of the medicinal form of the agent according to the invention thus made it possible to treat chronic viral hepatitis C with intolerance of specific anti-viral treatment drugs, which is a demonstration of the anti-viral activity of the agent according to the invention.

## Claims

1. A combination of bis-(γ-L-glutamyl)-L-cysteinyl-glycine or a salt thereof, inosine and coordination compounds formed by palladium, copper and (γ-L-glutamyl)-L-cysteinyl-glycine.

2. The combination as claimed in claim 1, in which the bis-(γ-L-glutamyl)-L-cysteinyl-glycine is in the form of the disodium salt.

3. The combination as claimed in claim 1, where the bis-(γ-L-glutamyl)-L-cysteinyl-glycine disodium salt:inosine:palladium:copper molar ratio is in the range 100-10000:100-10000:1-10:1-10.

4. The combination as claimed in claim 1, where the bis-(γ-L-glutamyl)-L-cysteinyl-glycine disodium salt:inosine:palladium:copper molar ratio is 1000:1000:1:1.

5. The combination as claimed in claim 1, where cis-diaminodichloropalladium is selected as the source of palladium.

6. The combination as claimed in claim 1, where copper dichloride is selected as the source of copper.

7. The combination as claimed in claims 1-6, which comprises a catalyst based on palladium cis-diaminodichloride, copper dichloride and (γ-L-glutamyl)-L-cysteinyl-glycine, prepared in situ.

8. The combination as claimed in claims 1-6, which comprises a catalyst based on palladium cis-diaminodichloride, copper dichloride and (γ-L-glutamyl)-L-cysteinyl-glycine, prepared in advance.

9. The combination as claimed in claim 1, which exhibits anti-viral activity.

10. The combination as claimed in claim 1, for use in the treatment of infectious, and in particular viral, diseases.

11. A pharmaceutical composition, which comprises the combination as claimed in any of claims 1-12 and a pharmaceutically acceptable excipient.

12. The pharmaceutical composition as claimed in claim 11, which exhibits anti-viral activity.

13. The pharmaceutical composition as claimed in claim 12, for use in the treatment of infectious, and in particular viral, diseases.

14. An anti-viral agent, which comprises a solution in acetate buffer of the combination as claimed in claim 1.

15. The agent as claimed in claim 14, where the bis-(γ-L-glutamyl)-L-cysteinyl-glycine disodium salt:inosine:palladium:copper molar ratio is in the range 100-10000:100-10000:1-10:1-10.

16. The agent as claimed in claim 15, where the bis-(γ-L-glutamyl)-L-cysteinyl-glycine disodium salt:inosine:palladium:copper molar ratio is 1000:1000:1:1.

17. The agent as claimed in claim 14, for the treatment of a virus selected from the group which includes influenza virus, hepatitis C virus, hepatitis B virus, tick-borne encephalitis virus, Rift Valley fever virus and Venezuelan equine encephalitis virus.

18. A method for the treatment of a viral disease in a patient in need thereof, in which an effective amount of the combination as claimed in any of claims 1-10, the composition as claimed in any of claims 11-13 or the agent as claimed in claims 14-17 is administered to the patient.

19. The method as claimed in claim 18, where the viral disease is selected from the group which includes influenza, hepatitis C, hepatitis B, tick-borne encephalitis, Rift Valley fever and Venezuelan equine encephalitis.
